# FASCICULE DE BREVET EUROPEEN

(11) **EP 0 836 863 B1**
(45) Date de publication et mention de la délivrance du brevet: **24.07.2002**
(21) Numéro de dépôt: 97402215.4
(22) Date de dépôt: 24.09.1997
(51) Int. Cl.: A61M 39/08, F16L 11/22, A61M 25/00

(54) **Cathéter avec corps souple à canaux multiples axiallement déchirable**
Axial aufreissbarer, mehrlumiger Katheterrumpf aus weichem Material
Axially tearable soft multilumen catheter body

(30) Priorité: 18.10.1996 FR 9612687
(43) Date de publication de la demande: 22.04.1998
(73) Titulaire: PORGES, 92350 Le Plessis-Robinson (FR)
(72) Inventeur: Cazal, Thierry, 24200 Sarlat (FR)
(74) Mandataire: Bonnetat, Christian

(56) Documents cités:
- EP-A- 0 332 366
- GB-A- 2 156 033
- US-A- 4 406 656
- US-A- 5 556 390

## Description

La présente invention concerne une sonde ou cathéther généralement utilisée en médecine ou en chirurgie, notamment en urodynamique ou dans le domaine cardiovasculaire. De telles sondes sont utilisées pour la mesure de pression et de débit ou pour la transmission de liquides, par exemple dans la vessie, l'urètre, etc ...

De façon générale, la présente invention se rapporte à une sonde médicale ou chirurgicale de type connu, destinée à être introduite dans un passage corporel et pourvue d'au moins deux canaux longitudinaux individuels s'étendant, chacun, entre l'extrémité proximale et l'extrémité distale de ladite sonde, cette sonde comportant :
- un corps allongé souple destiné à être introduit par son extrémité distale dans ledit passage corporel et pourvu d'au moins deux conduits longitudinaux internes ; et
- au moins deux branches proximales, allongées et souples, pourvues chacune d'un conduit longitudinal interne et prévues à l'extrémité proximale dudit corps allongé, chacun des conduits desdites branches prolongeant un conduit dudit corps allongé pour former l'un desdits canaux longitudinaux individuels de ladite sonde.

Ainsi, chacun desdits canaux, qui débouche à l'extérieur de la sonde aux extrémités proximale et distale de celle-ci, soit à travers des orifices latéraux, soit à travers des orifices d'extrémité, peut être utilisé pour la transmission de fluide et/ou de pression.

La réalisation de telles sondes est complexe et coûteuse. En effet, il est nécessaire de solidariser lesdites branches entre elles et avec ledit corps allongé souple, de façon que le conduit de chaque branche soit en continuité de communication étanche avec un conduit dudit corps allongé souple et avec ce seul conduit.

Cela entraîne des opérations de collage, soudage ou surmoulage complexes. II en résulte que le coût de telles sondes connues est élevé, et d'autant plus élevé que le nombre de canaux longitudinaux est plus grand.

Par ailleurs, le document EP-A-0 332 366 décrit un dispositif de drainage comportant au moins deux conduits distaux individuels reliés en commun, par un collecteur, à un unique conduit de drainage commun menant à l'extrémité proximale dudit dispositif de drainage, lesdits conduits distaux étant obtenus par séparation longitudinale d'un profilé multi-conduit dans lequel lesdits conduits sont juxtaposés et reliés l'un à l'autre par une paroi longitudinale déchirable. Pour faciliter la séparation desdits conduits distaux, une fente interne est pratiquée dans cette paroi longitudinale déchirable de manière à ne laisser subsister que deux lignes de liaison déchirables, en périphérie de ladite paroi de liaison.

Ainsi, ce dispositif de drainage connu n'est pas constitué d'une pièce unique entre son extrémité proximale et son extrémité distale. Au contraire, il est formé par l'assemblage de composants indépendants, à savoir les conduits distaux, le collecteur et le conduit proximal commun. On notera qu'un tel assemblage ne soulève d'ailleurs aucune difficulté, même si le nombre de conduits distaux est élevé, puisque chaque conduit distal individuel n'a pas à s'étendre sur la totalité du dispositif de drainage, mais seulement entre l'extrémité distale de celui-ci et ledit collecteur et puisque les conduits distaux individuels sont reliés en commun au conduit de drainage commun par le collecteur. II n'y a donc pas à relier de façon étendue et spécifique chaque conduit distal à un autre conduit individuel. Par ailleurs, il est évident que, de par sa forme aplatie, la fente interne à ladite paroi longitudinale déchirable ne saurait participer au drainage dans des conditions comparables à celles desdits conduits distaux.

La présente invention a pour objet une sonde qui, tout en pouvant être réalisée à faible coût, permet d'obtenir aisément un conduit longitudinal supplémentaire, par rapport au nombre des branches.

A cette fin, selon l'invention, la sonde médicale ou chirurgicale, destinée à être introduite dans un passage corporel et pourvue d'au moins deux canaux longitudinaux individuels s'étendant, chacun, entre l'extrémité proximale et l'extrémité distale de ladite sonde, cette sonde comportant :
- un corps allongé souple destiné à être introduit par son extrémité distale dans ledit passage corporel et pourvu d'au moins deux conduits longitudinaux internes, ledit corps allongé souple comportant, en plus desdits conduits longitudinaux internes, un canal longitudinal central délimitant dans ledit corps des portions longitudinales reliées entre elles par des lignes de matière superficielles, facilement déchirables; et
- au moins deux branches proximales, allongées et souples, pourvues chacune d'un conduit longitudinal interne et prévues à l'extrémité proximale dudit corps allongé, chacun des conduits desdites branches prolongeant un conduit dudit corps allongé pour former l'un desdits canaux longitudinaux individuels de ladite sonde, chacune desdites portions longitudinales du corps comportant un desdits conduits longitudinaux internes et chacune desdites branches proximales étant formée par une telle portion longitudinale dudit corps, séparée desdites autres portions longitudinales, sur une longueur limitée, le long desdites lignes de matière superficielles déchirables,
est caractérisée en ce qu'elle comporte un élément tubulaire rapportable audit corps allongé et en ce que ledit canal longitudinal central présente une section apte à recevoir ledit élément tubulaire, à l'embranchement desdites branches proximales de ladite sonde.

Ainsi, chacune desdites branches proximales peut être formée par séparation desdites portions longitudinales, à la manière du pelage d'une banane. De ce fait, chacune des branches proximales est constituée par une portion longitudinale du corps allongé souple et chaque conduit de ce dernier constitue le conduit d'une branche proximale. Par construction, chaque conduit d'une branche proximale est donc le prolongement d'un conduit spécifique du corps allongé souple pour former un canal longitudinal individuel. Il n'y a donc plus à procéder au raccord des branches proximales avec le corps et au raccord des conduits desdites branches avec les conduits dudit corps. On comprendra aisément que ledit corps peut être réalisé, à faible coût, par extrusion de matière synthétique.

Par ailleurs, dans la partie du corps où lesdites portions longitudinales ne sont pas séparées les unes des autres pour former lesdites branches proximales, on conçoit aisément que ledit canal longitudinal central peut être utilisé comme un canal longitudinal individuel supplémentaire, éventuellement prolongé, du côté proximal de la sonde, par ledit élément tubulaire.

De préférence, afin d'éviter que la séparation desdites branches proximales de la sonde conforme à l'invention se propage de façon intempestive le long dudit corps, lesdites branches proximales sont solidarisées les unes des autres à leur embranchement, par des moyens de solidarisation, par exemple par collage ou par pose d'une bague.

De tels moyens de solidarisation peuvent, de plus, solidariser ledit élément tubulaire à ladite sonde.

Comme on le verra ci-après, en regard des figures, ledit corps allongé peut comporter un nombre de portions longitudinales supérieur à deux, par exemple égal à trois ou quatre. Suivant le nombre desdites portions longitudinales, la section dudit canal longitudinal central peut présenter alors la forme approximative d'une lentille, d'un triangle, d'un carré, respectivement.

Les figures du dessin annexé feront bien comprendre comment l'invention peut être réalisée. Sur ces figures, des références identiques désignent des éléments semblables.

La figure 1 montre de façon schématique et simplifiée une sonde conforme à la présente invention.

La figure 2 est une coupe suivant la ligne II-II de la figure 1.

La figure 3 illustre, en vue semblable à la figure 1, une variante de réalisation de la sonde conforme à la présente invention.

La figure 4 est une coupe suivant la ligne IV-IV de la figure 3, au niveau de l'embranchement des branches sur le corps allongé.

La figure 5 montre, en coupe, une variante du corps de sonde conforme à la présente invention, ce corps comprenant trois portions longitudinales.

La figure 6 montre, également en coupe, une autre variante pour le corps allongé de la sonde conforme à la présente invention, ce corps comprenant quatre portions longitudinales.

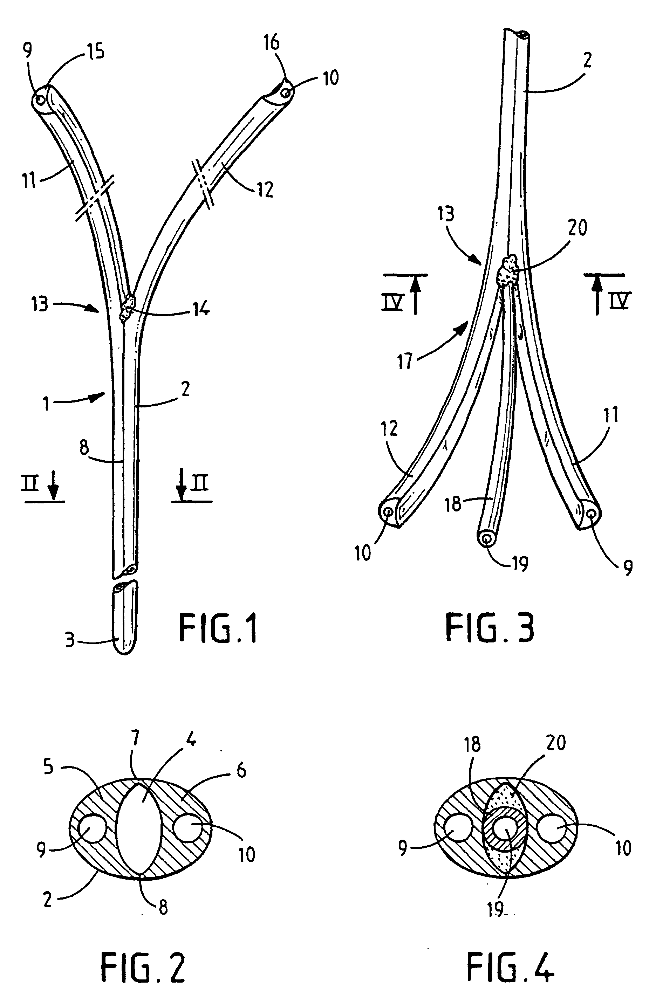

La sonde 1 montrée schématiquement par les figures 1 et 2 comporte un corps allongé souple 2 destiné à être introduit par son extrémité distale 3 dans un passage corporel. Comme le montre plus particulièrement la figure 2, le corps allongé 2 comporte un canal longitudinal central 4 délimitant dans ledit corps des portions longitudinales 5 et 6 reliées entre elles par des lignes de matière superficielles 7 et 8, facilement déchirables. Dans l'exemple representé, le canal longitudinal 4 présente la forme d'un lentille biconvexe, tandis que lesdites portions longitudinales 5, 6 ont la forme de croissants reliés par leurs cornes le long des lignes déchirables 7 et 8. Chacune desdites portions longitudinales 5 et 6 comporte un conduit longitudinal interne 9 ou 10.

Du côté de son extrémité proximale, la sonde comporte deux branches 11 et 12, respectivement constituées par les parties proximales des portions longitudinales 5 et 6, séparées l'une de l'autre par déchirement des lignes 7 et 8 sur une longueur limitée.

A l'embranchement 13 des branches proximales 11 et 12 sur le corps allongé 2, lesdites branches 11 et 12 sont solidarisées l'une de l'autre, par exemple par de la colle 14.

Bien entendu, de façon usuelle, les extrémités proximales 15 et 16 des branches 11 et 12 peuvent être pourvues de canules non représentées. On remarquera que les conduits longitudinaux internes 9 et 10 forment des canaux individuels s'étendant, chacun, entre l'extrémité proximale et l'extrémité distale de la sonde 1.

Comme l'illustrent les figures 3 et 4, on peut obtenir, à partir de la sonde 1 des figures 1 et 2, une sonde 17 à trois branches. La troisième branche est formée par un élément tubulaire 18 dont une extrémité est enfoncée dans le canal 4, au niveau de l'embranchement 13. Ainsi, le conduit 19 de l'élément tubulaire 18 est en communication fluide avec ledit canal longitudinal central 4. L'élément tubulaire 18 peut être fixé au corps 2 par de la colle 20 solidarisant sa paroi extérieure à la paroi du canal longitudinal 4. Dans ce cas, la colle 20 solidarise de plus les bandes proximales 11 et 12 entre elles.

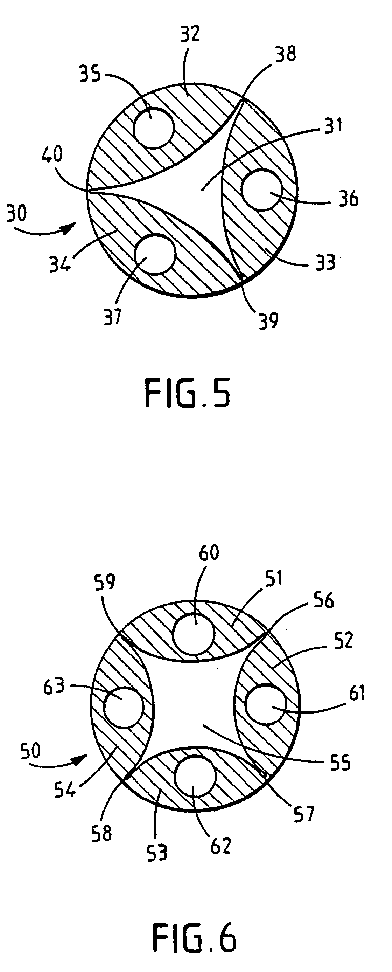

Les figures 5 et 6 illustrent, en section comparable à la figure 2, des variantes de réalisation de la sonde conforme à la présente invention.

Sur la figure 5, on a représenté un corps 30 (semblable au corps 2) comportant un canal longitudinal central 31 (comparable au canal longitudinal central 4) délimitant dans ledit corps 30 trois portions longitudinales 32, 33 et 34 (comparables aux portions longitudinales 5 et 6), chacune étant pourvue d'un conduit longitudinal interne 35, 36 et 37 (comparables aux conduits 9 et 10).

Dans ce mode de réalisation, le canal longitudinal central 31 présente en section la forme d'un triangle à côtés curvilignes concaves, tandis que les portions longitudinales 32, 33 et 34 présentent la forme de lentilles biconvexes reliées entre elles par des lignes de matière superficielles, facilement déchirables 38, 39 et 40. On comprendra aisément que le corps allongé souple 30 peut facilement former trois branches (comparables aux branches 11 et 12), par séparation desdites portions longitudinales 32, 33 et 34 le long d'une partie limitée des lignes 38, 39 et 40, à l'extrémité proximale de la sonde 30.

Dans le mode de réalisation 50 de la figure 6, on prévoit quatre portions longitudinales 51, 52, 53 et 54 pouvant donner naissance à quatre branches semblables aux branches 11 et 12.

Dans ce mode de réalisation 50, le canal longitudinal central 55 présente la forme d'un carré à côtés curvilignes concaves, tandis que chacune des portions 51, 52, 53 et 54 présente la forme d'une lentille biconvexe, ces lentilles étant reliées les unes aux autres par des lignes superficielles facilement déchirables 56, 57, 58 et. 59. Les portions longitudinales 51 à 54 comportent chacune un conduit longitudinal 60 à 63.

Bien entendu, la sonde constituée par le corps 50 peut comporter quatre branches semblables aux branches 11 et 12, respectivement constituées par séparation, sur une longueur limitée, des portions longitudinales 51, 52, 53 et 54 le long des lignes 56 à 59.

On remarquera que, dans les modes de réalisation des figures 5 et 6, il est possible de prévoir une branche supplémentaire, en introduisant un élément tubulaire 18 dans le canal longitudinal central 31 ou 55.

On notera par ailleurs que les corps allongés souples 2, 30 et 50 peuvent facilement être obtenus, à faible coût, par extrusion d'une matière synthétique.

## Revendications

1. Sonde médicale ou chirurgicale, destinée à être introduite dans un passage corporel et pourvue d'au moins deux canaux longitudinaux individuels s'étendant, chacun, entre l'extrémité proximale et l'extrémité distale de ladite sonde, cette sonde comportant :
- un corps allongé souple (2, 30, 50) destiné à être introduit par son extrémité distale (3) dans ledit passage corporel et pourvu d'au moins deux conduits longitudinaux internes (9, 10 ; 35, 36, 37; 60, 61, 62, 63), ledit corps allongé souple (2, 30, 50) comportant, en plus desdits conduits longitudinaux internes, un canal longitudinal central (4, 31, 55) délimitant dans ledit corps des portions longitudinales (5, 6 ; 32, 33, 34 ; 51, 52, 53, 54) reliées entre elles par des lignes de matière superficielles, facilement déchirables; et
- au moins deux branches proximales (11, 12), allongées et souples, pourvues chacune d'un conduit longitudinal interne et prévues à l'extrémité proximale dudit corps allongé, chacun des conduits desdites branches prolongeant un conduit dudit corps allongé pour former l'un desdits canaux longitudinaux individuels de ladite sonde, chacune desdites portions longitudinales du corps comportant un desdits conduits longitudinaux internes (9, 10 ; 35, 36, 37 ; 60, 61, 62, 63) et chacune desdites branches proximales étant formée par une telle portion longitudinale dudit corps, séparée desdites autres portions longitudinales, sur une longueur limitée, le long desdites lignes de matière superficielles déchirables,
**caractérisée en ce qu'**elle comporte un élément tubulaire (18) rapportable audit corps allongé et **en ce que** ledit canal longitudinal central (4, 31, 55) présente une section apte à recevoir ledit élément tubulaire (18), à l'embranchement (13) desdites branches proximales (11, 12) de ladite sonde.

2. Sonde selon la revendication 1,
**caractérisée en ce que** lesdites branches proximales (11, 12) sont solidarisées les unes des autres, à leur embranchement (13), par des moyens de solidarisation (14, 20).

3. Sonde selon la revendication 2,
**caractérisée en ce que** lesdits moyens de solidarisation (20) solidarisent ledit élément tubulaire (18) à ladite sonde.

4. Sonde selon l'une des revendications 1 à 3,
**caractérisée en ce que** ledit corps allongé souple (2) comporte deux portions longitudinales (5, 6) et **en ce que** ledit canal longitudinal central (4) a une section en forme approximative de lentille.

5. Sonde selon l'une des revendications 1 à 3,
**caractérisée en ce que** ledit corps allongé souple (30) comporte trois portions longitudinales (32, 33, 34) et **en ce que** ledit canal longitudinal central (31) a une section en forme approximative de triangle.

6. Sonde selon l'une des revendications 1 à 3,
**caractérisée en ce que** ledit corps allongé souple (50) comporte quatre portions longitudinales (51, 52, 53, 54) et **en ce que** ledit canal longitudinal (55) a une section en forme approximative de carré.

## Patentansprüche

1. Medizinische oder chirurgische Sonde zur Einführung in einen Körpergang, die mit mindestens zwei einzelnen Längskanälen versehen ist, die sich jeweils zwischen dem proximalen und dem distalen Ende der Sonde erstrecken, wobei die Sonde Folgendes umfasst:
• einen länglichen flexiblen Körper (2, 30, 50), der über sein distales Ende (3) in den Körpergang eingeführt werden soll und mit mindestens zwei internen Längsleitungen (9, 10; 35, 36, 37; 60, 61, 62, 63) versehen ist sowie außer diesen internen Längsleitungen einen mittleren Längskanal (4, 31, 55) umfasst, der in dem Körper Längsabschnitte (5, 6; 32, 33, 34; 51, 53, 54) begrenzt, die durch leicht zerreißbare Oberflächenmateriallinien miteinander verbunden sind, und
• mindestens zwei längliche und flexible proximale Schenkel (11, 12), die jeweils mit einer internen Längsleitung versehen und am proximalen Ende des länglichen Körpers vorgesehen sind, wobei jede der Leitungen der Schenkel unter Bildung einer der einzelnen Längskanäle der Sonde eine Verlängerung einer Leitung des länglichen Körpers darstellt, wobei jeder der Längsabschnitte des Körpers eine der internen Längsleitungen (9, 10; 35, 36, 37; 60, 61, 62, 63) umfasst und jeder der proximalen Schenkel von einem solchen Längsabschnitt des Körpers gebildet wird, der über eine begrenzte Länge entlang der zerreißbaren Oberflächenmateriallinien von den anderen Längsabschnitten getrennt ist,
**dadurch gekennzeichnet, dass** sie ein an den länglichen Körper anfügbares rohrförmiges Element (18) umfasst und dass der Querschnitt des mittleren Längskanals (4, 31, 55) groß genug ist, um das rohrförmige Element (18) an der Verzweigung (13) der proximalen Schenkel (11, 12) der Sonde aufzunehmen.

2. Sonde nach Anspruch 1, **dadurch gekennzeichnet, dass** die proximalen Schenkel (11, 12) an ihrer Verzweigung (13) durch Verbindungsmittel (14, 20) miteinander verbunden sind.

3. Sonde nach Anspruch 2, **dadurch gekennzeichnet, dass** die Verbindungsmittel (20) das rohrförmige Element (18) mit der Sonde verbinden.

4. Sonde nach einem der Ansprüche 1 bis 3, **dadurch gekennzeichnet, dass** der flexible längliche Körper (2) zwei Längsabschnitte (5, 6) umfasst und dass der Querschnitt des mittleren Längskanals (4) etwa linsenförmig ist.

5. Sonde nach einem der Ansprüche 1 bis 3, **dadurch gekennzeichnet, dass** der flexible längliche Körper (30) drei Längsabschnitte (32, 33, 34) umfasst und dass der Querschnitt des mittleren Längskanals (31) etwa dreieckig ist.

6. Sonde nach einem der Ansprüche 1 bis 3, **dadurch gekennzeichnet, dass** der flexible längliche Körper (50) vier Längsabschnitte (51, 52, 53, 54) umfasst und dass der Querschnitt des mittleren Längskanals (55) etwa quadratisch ist.

## Claims

1. Medical or surgical probe, intended to be introduced into a body passage and provided with at least two individual longitudinal channels, each extending between the proximal end and the distal end of the said probe, this probe including:
- a flexible and elongate body (2, 30, 50) intended to be introduced via its distal end (3) into the said body passage and provided with at least two internal longitudinal conduits (9, 10; 35, 36, 37; 60, 61, 62, 63) the said flexible and elongate body (2, 30, 50) including, in addition to the said internal longitudinal conduits, a central longitudinal channel (4, 31, 55) delimiting, within the said body, longitudinal portions (5, 6; 32, 33, 34; 51, 52, 53, 54) connected to one another by surface material lines, which are easily tearable; and
- at least two flexible and elongate proximal branches (11, 12) which are each provided with an internal longitudinal conduit and are arranged at the proximal end of the said elongate body, each of the conduits of the said branches continuing a conduit of the said elongate body in order to form one of the said individual longitudinal channels of the said probe,
each of the said longitudinal portions of the body including one of the said internal longitudinal conduits (9, 10; 35, 36, 37; 60, 61, 62, 63) and
each of the said proximal branches being formed by one such longitudinal portion of the said body, separated from the said other longitudinal portions, over a limited length, along the said tearable surface material lines,
**characterized in that** it includes a tubular element (18) which can be attached to the said elongate body and **in that** the said central longitudinal channel (4, 31, 55) has a cross section which is capable of receiving the said tubular element (18) at the junction (13) of the said proximal branches (11, 12) of the said probe.

2. Probe according to Claim 1, **characterized in that** the said proximal branches (11, 12) are joined to one another, at their junction (13), by joining means (14, 20).

3. Probe according to Claim 2, **characterized in that** the said joining means (20) join the said tubular element (18) to the said probe.

4. Probe according to one of Claims 1 to 3, **characterized in that** the said flexible and elongate body (2) includes two longitudinal portions (5, 6), and **in that** the said central longitudinal channel (4) has a cross section in the approximate shape of a lens.

5. Probe according to one of Claims 1 to 3, **characterized in that** the said flexible and elongate body (30) includes three longitudinal portions (32, 33, 34), and **in that** the said central longitudinal channel (31) has a cross section in the approximate shape of a triangle.

6. Probe according to one of Claims 1 to 3, **characterized in that** the said flexible and elongate body (50) includes four longitudinal portions (51, 52, 53, 54), and **in that** said longitudinal channel (55) has a cross section in the approximate shape of a square.
